(19) Europäisches Patentamt
European Patent Office
Office européen des brevets

(11) EP 0 674 645 B1

(12) EUROPEAN PATENT SPECIFICATION

(45) Date of publication and mention of the grant of the patent:
16.07.1997 Bulletin 1997/29

(21) Application number: 94903000.1

(22) Date of filing: 15.12.1993

(51) Int Cl.[6]: C07D 501/48, A61K 31/545

(86) International application number:
PCT/JP93/01814

(87) International publication number:
WO 94/14818 (07.07.1994 Gazette 1994/15)

# (54) CEPHEM COMPOUNDS WITH ANTIMICROBIAL ACTIVITY

CEPHEM VERBINDUNGEN MIT ANTIMICROBIZIDE EIGENSCHAFTEN

COMPOSES DE CEPHEME A ACTIVITE ANTIMICROBIENNE

(84) Designated Contracting States:
AT BE CH DE DK ES FR GB GR IE IT LI LU NL PT SE

(30) Priority: 21.12.1992 US 993615

(43) Date of publication of application:
04.10.1995 Bulletin 1995/40

(73) Proprietor: FUJISAWA PHARMACEUTICAL CO., LTD.
Osaka-shi Osaka 541 (JP)

(72) Inventors:
- TAKAYA, Takao
Kawanishi-shi, Hyogo 666-01 (JP)
- SAKANE, Kazuo
Kawanishi-shi,Hyogo 666-01 (JP)
- MIYAI, Kenzi
Kawanishi-shi, Hyogo 666 (JP)
- KAWABATA, Kohji
Kawanishi-shi, Hyogo 666-01 (JP)
- OKUDA, Shinya
Kawanishi-shi, Hyogo 666-01 (JP)
- OHKI, Hidenori
Ikeda-shi, Osaka 563 (JP)
- YAMANAKA, Hideaki
Hirakata-shi, Osaka 573 (JP)

(74) Representative: Hrabal, Ulrich, Dr. et al
Türk, Gille, Hrabal, Leifert
Brucknerstrasse 20
40593 Düsseldorf (DE)

(56) References cited:
EP-A- 0 027 599
EP-A- 0 042 154
EP-A- 0 238 060
EP-A- 0 306 863

- American Journal of Med., Vol. 94, pp313-328
- British Med. Journal, Vol. 307, pp1049-1052

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 674 645 B1

## Description

Technical Field

The present invention relates to a new cephem compound and pharmaceutically acceptable salts thereof, which have antimicrobial activities, to pharmaceutical composition comprising the same, and to a use of said compound for the manufacture of a medicament for preventing and/or treating infectious diseases in human beings and animals.

Accordingly, one object of the present invention is to provide the cephem compound and pharmaceutically acceptable salts thereof, which is highly active against a number of pathogenic microorganisms.

A further object of the present invention is to provide pharmaceutical composition comprising, as an active ingredient, said cephem compound or its pharmaceutically acceptable salts.

Background Art

Cephem compounds having high antimicrobial activity against wide variety of pathogenic microorganisms including Gram-positive and Gram-negative microorganisms have been known in, for example, EP-A-306863, but they are still insufficient as antimicrobial agents against methicillin resistant Staphylococcus aureus (MRSA) and Pseudomonas aeruginosa.

Disclosure of the Invention

The object cephem compound of the present invention is novel and can be represented by the following formula [I] :

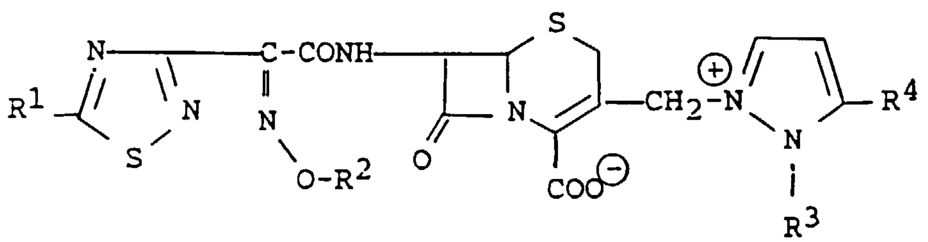

[I]

wherein

$R^1$ is amino
$R^2$ is ethyl
$R^3$ is hydroxyethyl, and
$R^4$ is amino.

As to the object compound [I], the following points are to be noted.

That is, the partial structure of the formula :

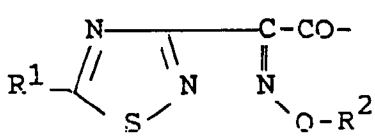

(wherein $R^1$ and $R^2$ are each as defined above) shows that the object compound [I] and the starting compound [III] mentioned below are syn isomers.

Another point to be noted is that the pyrazolio moiety of the compound [I] can also exist in the tautomeric form, and such tautomeric equilibrium can be represented by the following schemes.

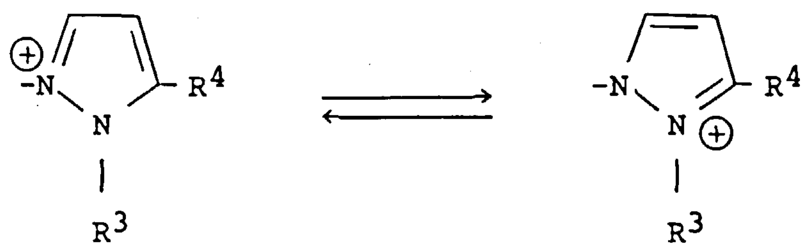

(A) (B)

(wherein $R^3$ and $R^4$ are each as defined above).

Both of the above tautomeric isomers are included within the scope of the present invention, and in the present specification and claim, however, the object compound [I] is represented for the convenient sake by one expression of the pyrazolio group of the formula (A).

The cephem compound [I] or the present invention can be prepared by a process as illustrated in the following.

Process 1

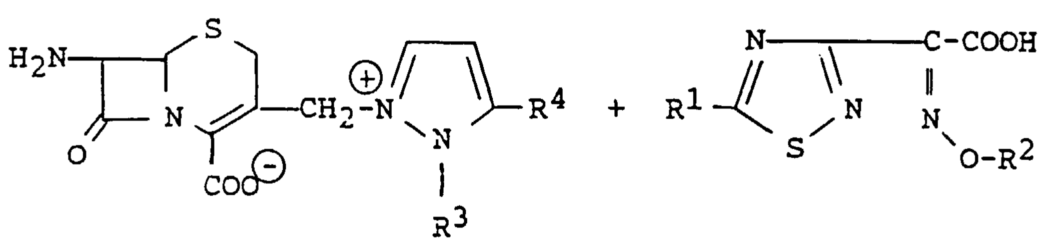

[II]
or its reactive derivative at the amino group
or a salt thereof

[III]
or its reactive derivative at the carboxy group
or a salt thereof

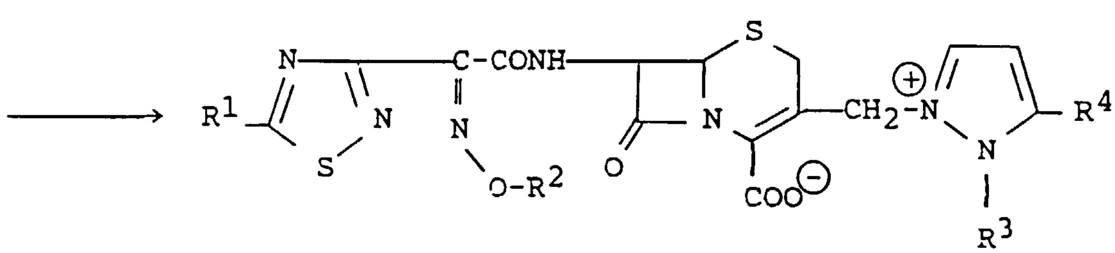

[I]
or its salt

wherein $R^1$, $R^2$, $R^3$ and $R^4$ are each as defined above.

In the above and subsequent descriptions of this specification, suitable examples of the various definitions are

explained in detail as follows.

Suitable pharmaceutically acceptable salts of the object compound [I] are conventional non-toxic salts and include an acid addition salt such as an organic acid addition salt [e.g. formate, acetate, trifluoroacetate, maleate, tartrate, methanesulfonate, benzenesulfonate, toluenesulfonate, etc.], an inorganic acid addition salt [e.g. hydrochloride, hydrobromide, sulfate, phosphate, etc.], a salt with an amino acid [e.g. arginine salt, aspartic acid salt, glutamic acid salt, etc.], and the like.

The process for preparing the object compound of the present invention is explained in detail in the following.

Process 1

The compound [I] or its salt can be prepared by reacting the compound [II] or its reactive derivative at the amino group or a salt thereof with the compound [III] or its reactive derivative at the carboxy group or a salt thereof.

Suitable reactive derivative at the amino group of the compound [II] may include Schiff's base type imino or its tautomeric enamine type isomer formed by the reaction of the compound [II] with a carbonyl compound such as aldehyde, ketone or the like; a silyl derivative formed by the reaction of the compound [II] with a silyl compound such as bis(trimethylsilyl)acetamide,
mono(trimethylsilyl)acetamide [e.g. N-(trimethylsilyl)acetamide], bis(trimethylsilyl)urea or the like; a derivative formed by reaction of the compound [II] with phosphorus trichloride or phosgene, and the like.

Suitable salts of the compound [II] and its reactive derivative can be referred to the ones as exemplified for the compound [I].

Suitable reactive derivative at the carboxy group of the compound [III] may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid, etc.], dialkylphosphorous acid, sulfurous acid, thiosulfuric acid, sulfuric acid, sulfonic acid [e.g. methanesulfonic acid, etc.], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid, etc.] or aromatic carboxylic acid [e.g. benzoic acid, etc.]; a symmetrical acid anhydride; an activated amide with imidazole, 4-substituted imidazole, dimethylpyrazole, triazole or tetrazole; or an activated ester [e.g. cyanomethyl ester, methoxymethyl ester, dimethyliminomethyl [$(CH_3)_2N^+$=CH-] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioeter, p-cresyl thioester, carboxymethyl thioester, pyranyl ester, pyridyl ester, piperidyl ester, 8-quinolyl thioester, etc.], or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-lH-benzotriazole, etc.], and the like. These reactive derivatives can optionally be selected from them according to the kind of the compound [III] to be used.

Suitable salts of the compound [III] and its reactive derivative can be referred to the ones as exemplified for the compound [I].

The reaction is usually carried out in a conventional solvent such as water, alcohol [e.g. methanol, ethanol, etc.], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvents may also be used in a mixture with water.

In this reaction, when the compound [III] is used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide, N,N'-diisopropylcarbodiimide; N-ethyl-N'-(3-dimethylaminopropyl)carbodiimide; N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus pentachloride; phosphorus trichloride; thionyl chloride; oxalyl chloride; lower alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, phosphorus oxychloride, etc.; or the like.

The reaction may also be carried out in the presence of an inorganic or organic base such as an alkali metal bicarbonate, tri(lower)alkylamine, pyridine, N-(lower)alkylmorpholine, N,N-di(lower)alkylbenzylamine, or the like.

The reaction temperature is not critical, and the reaction is usually carried out under cooling to warming.

The object compound [I] and its pharmaceutically acceptable salt are novel and exhibit high antimicrobial activity, inhibiting the growth of a wide variety of pathogenic microorganisms including Gram-positive and Gram-negative microorganisms, especially, MRSA and Pseudomonas aeruginosa, and are useful as antimicrobial agents.

Now in order to show the utility of the object compound [I], the test data on MIC (minimal inhibitory concentration) of a representative compound of this invention are shown in the following.

Test method :

In vitro antibacterial activity was determined by the two-fold agar-plate dilution method as described below.

One loopful of an overnight culture of each test strain in Trypticase-soy broth ($10^6$ viable cells per ml) was streaked on heart infusion agar (HI-agar) containing graded concentrations of representative test compound, and the minimal inhibitory concentration (MIC) was expressed in terms of μg/ml after incubation at 37°C for 20 hours.

Test compound :

7β-[2-(5-Amino-1,2,4-thiadiazol-3-yl)-2-ethoxyiminoacetamido]-3-[3-amino-2-(2-hydroxyethyl)-1-pyrazolio]methyl-3-cephem-4-carboxylate hydrochloride (syn isomer)

Test results :

| Test Strain | MIC (μg/ml) |
|---|---|
| S. aureus 2538 | 3.13 |
| E. coli 31 | ≦0.025 |
| P. aeruginosa 2 | 0.39 |

For therapeutic administration, the object compound [I] and its pharmaceutically acceptable salt of the present invention are used in the form of conventional pharmaceutical preparation which contains said compound as an active ingredient, in admixture with pharmaceutically acceptable carriers such as an organic or inorganic solid or liquid excipient which is suitable for oral, parenteral such as intravenous, intramuscular, subcutaneous or intraarticular, and external (topical)administration. The pharmaceutical preparations may be in solid form such as tablet, granule, powder, capsule, suppository, or liquid form such as solution, suspension, syrup, emulsion, lemonade and the like.

If needed, there may be included in the above preparations auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, arginine, sodium bicarbonate, sodium carbonate, and the like.

While the dosage of the compound [I] may vary from and also depend upon the age, conditions of the patient, a kind of diseases, etc. In general amounts between 1 mg and about 4,000 mg or even more per day may be administered to a patient. An average single dose of about 50 mg, 100 mg, 250 mg, 500 mg, 1000 mg, 2000 mg of the object compound [I] of the present invention may be used in preventing and/or treating diseases infected by pathogenic microorganisms.

Examples

The following Preparations and Examples are given for the purpose of illustrating the present invention in more detail.

Example 1

A solution of 7β-amino-3-[3-amino-2-(2-hydroxyethyl)-1-pyrazolio]methyl-3-cephem-4-carboxylate hydrochloride (50 g) in a mixture of acetonitrile (500 ml) and water (500 ml) was adjusted to pH 6.0 with aqueous sodium bicarbonate solution. To the resulting solution was added (Z)-2-(5-amino-1,2,4-thiadiazol-3-yl)-2-ethoxyiminoacetyl chloride hydrochloride (40 g) under stirring at 5°C and the mixture was stirred at the same temperature for 1.5 hours, keeping pH 5.0-6.5 with aqueous sodium bicarbonate solution. The reaction mixture was evaporated to remove the containing organic solvent. The aqueous solution was subjected to column chromatography on Sepabeads SP-205 (Trademark : Mitsubishi Kasei Corporation) (1.3 ℓ). The column was washed with water (5 ℓ) and the object compound was eluted with 15% isopropyl alcohol, and the eluate was lyophilized to give 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate (syn isomer) (51.2 g).

IR (Nujol) : 3300, 1750, 1660, 1600 $cm^{-1}$
NMR ($DMSO-d_6$, δ) : 1.23 (3H, t, J=7Hz), 2.93 and 3.18 (2H, ABq, J=18Hz), 3.58 (2H, br s), 4.15 (2H, q, J=7Hz), 4.13-4.25 (2H, m), 5.01 (1H, d, J=5Hz), 5.03 and 5.23 (2H, ABq, J=16Hz), 5.63 (1H, dd, J=5Hz, 8Hz), 5.81 (1H, d, J=3Hz), 7.27 (2H, br s), 8.10 (IH, d, J=3Hz), 8.15 (2H, br s), 9.50 (IH, d, J=8Hz)

Example 2

To a solution of 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate (syn isomer) (30 g) in 4N-hydrochloric acid (22.4 ml) was added acetone (120 ml) under stirring at 20°C. The stirring was continued for 10 hours at the same temperature to give crystals, which were collected by filtration and dried to give 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate hydrochloride (syn isomer) (13.46 g).

IR (Nujol) : 3250, 3150, 3120, 1785, 1705, 1650, 1620, 1575, 1530 $cm^{-1}$
NMR ($DMSO-d_6$, δ) : 1.23 (3H, t, J=7Hz), 3.24 and 3.30 (2H, ABq, J=18Hz), 3.60 (2H, br s), 4.03-4.25 (2H, m), 4.13 (2H, q, J=7Hz), 5.14 and 5.32 (2H, d, J=16Hz), 5.18 (1H, d, J=5Hz), 5.86 (1H, dd, J=5Hz, 8Hz), 5.89 (IH, d, J=3Hz), 7.50 (2H, br s), 8.03 (1H, d, J=3Hz), 8.21 (2H, br s), 8.58 (1H, d, J=8Hz)

Example 3

To a solution of 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate (syn isomer) (5 g) in water (50 ml) was added 1M sulfuric acid (8 ml). The solution was lyophilized to give 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate sulfate (syn isomer) (5.2 g).

IR (Nujol) : 3150, 1760, 1640, 1610 $cm^{-1}$
NMR ($DMSO-d_6$, δ) : 1.23 (3H, t, J=7Hz), 3.21 and 3.32 (2H, ABq, J=18Hz), 3.62 (2H, br s), 4.03-4.26 (2H, m), 4.13 (2H, q, J=7Hz), 5.88 (1H, dd, J=5Hz, 8Hz), 5.90 (1H, d, J=3Hz), 7.50 (2H, br s), 8.04 (1H, d, J=3Hz), 8.21 (2H, br s), 8.59 (1H, d, J=8Hz)

Example 4

Crystals of 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate sulfate (syn isomer) was obtained from 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate (syn isomer) and 2M sulfuric acid according to a similar manner to that of Example 2.

IR (KBr) : 3257, 3060, 1799, 1697, 1670, 1639, 1579, 1546 $cm^{-1}$
NMR ($D_2O$, δ) : 1.31 (3H, t, J=7Hz), 3.18 and 3.48 (2H, ABq, J=18Hz), 3.85 (2H, t, J=5Hz), 4.22-4.45 (4H, m), 5.20 (2H, s), 5.26 (1H, d, J=5Hz), 5.88 (1H, d, J=5Hz), 5.97 (1H, d, J=3Hz), 7.89 (1H, d, J=3Hz)

## Claims

1. 7β-[2-(5-amino-1,2,4-thiadiazol-3-yl)-2-ethoxyiminoacetamido]-3-[3-amino-2-(2-hydroxyethyl)-1-pyrazolio]methyl-3-cephem-4-carboxylate (syn isomer) or its acid addition salt.

2. A pharmaceutical composition which comprises, as an active ingredient, a compound of claim 1 or its pharmaceutically acceptable salt in admixture with pharmaceutically acceptable carriers.

3. A compound of claim 1 or its pharmaceutically acceptable salt for use as a medicament.

4. Use of a compound of claim 1 for manufacture of a medicament for the prevention and/or the treatment of infectious diseases.

## Patentansprüche

1. 7β-[2-(5-Amino-1,2,4-thiadiazol-3-yl)-2-ethoxyimino-acetamido]-3-[3-amino-2-(2-hydroxyethyl)-1-pyrazolio]methyl-3-cephem-4-Carboxylat (syn-Isomer) oder sein Säureadditionssalz.

2. Pharmazeutische Zusammensetzung, die als aktiven Bestandteil eine Verbindung nach Anspruch 1 oder ihr pharmazeutisch verträgliches Salz im Gemisch mit pharmazeutisch verträglichen Trägern umfaßt.

3. Verbindung nach Anspruch 1 oder ihr pharmazeutisch verträgliches Salz zur Verwendung als Medikament.

4. Verwendung einer Verbindung nach Anspruch 1 zur Herstellung eines Medikaments für die Prävention und/oder Behandlung von infektiösen Erkrankungen.

## Revendications

1. 7β-[2-(5-amino-1,2,4-thiadiazol-3-yl)-2-éthoxyiminoacétamido]-3-[3-amino-2-(2-hydroxyéthyl)-1-pyrazolio]méthyl-3-céphème-4-carboxylate (isomère syn) ou son sel d'addition avec un acide.

2. Composition pharmaceutique qui comprend, comme ingrédient actif, un composé selon la revendication 1 ou son sel pharmaceutiquement acceptable en mélange avec des supports pharmaceutiquement acceptables.

3. Composé selon la revendication 1 ou son sel pharmaceutiquement acceptable destiné à être utilisé comme médicament.

4. Utilisation d'un composé selon la revendication 1 pour la fabrication d'un médicament pour la prévention et/ou le traitement de maladies infectieuses.